(19) **Europäisches Patentamt / European Patent Office / Office européen des brevets**

(11) **EP 4 465 046 A2**

(12) # EUROPEAN PATENT APPLICATION

(43) Date of publication:
**20.11.2024 Bulletin 2024/47**

(51) International Patent Classification (IPC):
***G01N 33/543*** (2006.01)

(21) Application number: **24172838.5**

(52) Cooperative Patent Classification (CPC):
**G01N 33/54366**

(22) Date of filing: **26.04.2024**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023 US 202318309612**

(71) Applicant: **Analog Devices International Unlimited Company**
**Limerick, V94 RT99 (IE)**

(72) Inventors:
- **Sparks, Andrew**
  **Wilmington, 01887 (US)**
- **Berney, Helen**
  **Limerick, V94 RT99 (IE)**

(74) Representative: **Elkington and Fife LLP**
**Prospect House**
**8 Pembroke Road**
**Sevenoaks, Kent TN13 1XR (GB)**

(54) **SYSTEM, METHOD AND SENSOR ASSEMBLY**

(57) The present disclosure provides systems and methods for determining a property of target analyte in a sample. The systems and methods use a sensor assembly comprising plural sensing sites, each of the sensing sites comprising a capture species configured to specifically bind with the same target analyte. Each capture species is different and has a different binding affinity for the target analyte to the capture species on the other of the plural sensing sites.

FIG. 1

## Description

FIELD OF THE DISCLOSURE

This disclosure relates to systems, methods and a sensor assembly for determining a property of an analyte in a sample.

BACKGROUND

Various biological and chemical assay are known for sensing analytes. Analytes may, for example, include biomarkers, such as hormones, established to assist in patient monitoring and/or diagnosis.

Although rapid improvements in the selectivity and sensitivity of these sensors have been made over the past decades, there is still a need to improve the selectivity, sensitivity and speed of measurement, particularly for biological sensing systems. For example, often there is a trade-off between sensitivity and the range of concentrations that can be detected. A sensor which can measure across a broad range of concentrations will often lack accuracy. Conversely a sensor which is very accurate will often have a narrow dynamic range.

In biosensors and assays, such as standard enzyme-linked immunosorbent assays (ELISA), employed for quantifying analytes such as peptides, proteins, antibodies, and hormones, it has been found to be challenging to make quantitative measurements of analytes over a wide range of concentrations.

There is also a desire to obtain a measurement more quickly. Many sensors, particular biosensors, rely on the binding of a target analyte with a capture species. The rate determining step in obtaining a measurement is typically the time it takes for the target analyte to reach the capture species (e.g. due to flow and diffusion) and to bind to the capture species. Many sensors require an equilibrium to be established, which can take a relatively long time. It would be advantageous to provide a way of obtaining an accurate measurement quickly.

SUMMARY OF THE DISCLOSURE

The present disclosure provides systems and methods for determining a property of target analyte in a sample. The systems and methods use a sensor assembly comprising plural sensing sites, each of the sensing sites comprising a capture species configured to specifically bind with the same target analyte. Each capture species is different and has a different binding affinity for the target analyte to the capture species on the other of the plural sensing sites.

In one aspect, a system for determining a property of target analyte in a sample comprises a sensor assembly. The sensor assembly comprises plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; The system further comprises a control unit configured to determine the property of the target analyte, wherein the control unit is configured to use measurement signals from at least two of the plural sensing sites in determining the property of the target analyte.

In another aspect, a method for determining a property of target analyte in a sample comprises providing a sensor assembly, wherein the sensor assembly comprises comprising plural sensing sites, each of the sensing sites comprising a capture species configured to specifically bind with the target analyte and wherein each sensing site provides a measurement signal indicative of the interaction of the respective capture species with the target analyte. The capture species on each of the plural sensing sites is different to the capture species on the other of the plural sensing sites, and each capture species has a binding affinity for the target analyte, the binding affinity of each of the capture species being different to the other of the capture species. The method further comprises determining the property of the target analyte using measurement signals from at least two of the plural sensing sites.

In another aspect, a system for determining a property of target analyte in a sample comprises: a sensor assembly, comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species. The system further comprises a control unit configured to determine the property of the target analyte by: determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

In another aspect, a method for determining a property of target analyte in a sample comprises: providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species. The method further comprises determining the property of the target analyte by determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

In another aspect, a sensor assembly for a target analyte in a sample comprises plural sensing sites, each of the sensing sites comprising a capture species configured to specifically bind with a target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species.

BRIEF DESCRIPTION OF THE DRAWINGS

The present invention will now be described in more detail with reference to the accompanying drawings, which are not intended to be limiting:

Fig. 1 provides a schematic plan view of a system according to an embodiment;
Fig. 2A provides a schematic plan view of a sensor assembly for use in the system of Fig. 1 and according to an embodiment;
Fig. 2B provides a schematic cross-sectional view of the sensor assembly of Fig. 2A;
Fig. 3 provides a schematic graph plotting signal-to-noise ratio (SNR) against concentration for capture species having different affinities according to an embodiment;
Fig. 4 provides a schematic graph plotting signal-to-noise ratio (SNR) against concentration for capture species having different affinities according to an embodiment;
Fig. 5 provides a schematic plan view of a sensor assembly for use in the system of Fig. 1 and according to an example;
Fig. 6 provides a schematic graph plotting signal-to-noise ratio (SNR) against concentration for capture species having different affinities according to an embodiment;

Fig. 7 depicts a method according to an embodiment; and
Fig. 8 depicts a method according to an embodiment.

DETAILED DESCRIPTION

Various analyte sensing techniques are known. However, quantitative measurement of analytes over a wide range of concentrations is a challenge. Typically there is a trade-off between sensitivity and the range of concentrations that can be detected. It would also be advantageous to improve the speed of measurement.

In a first aspect, there is provided a system for determining a property of target analyte in a sample, the system comprising a sensor assembly. The sensor assembly comprises plural sensing sites, each of the sensing sites comprising a capture species configured to specifically bind with the target analyte and wherein each sensing site provides a measurement signal indicative of the interaction of the respective capture species with the target analyte. The capture species on each of the plural sensing sites is different to the capture species on the other of the plural sensing sites, and each capture species has a binding affinity ($K_a$) for the target analyte, the binding affinity of each of the capture species being different to the other of the capture species. The system further comprises a control unit configured to determine the property of the target analyte, wherein the control unit is configured to use measurement signals from at least two of the plural sensing sites in determining the property of the target analyte.

In a second aspect, there is provided method for determining a property of target analyte in a sample, the method comprising providing a sensor assembly, wherein the sensor assembly comprises comprising plural sensing sites, each of the sensing sites comprising a capture species configured to specifically bind with the target analyte and wherein each sensing site provides a measurement signal indicative of the interaction of the respective capture species with the target analyte. The capture species on each of the plural sensing sites is different to the capture species on the other of the plural sensing sites, and each capture species has a binding affinity for the target analyte, the binding affinity of each of the capture species being different to the other of the capture species. The method further comprises determining the property of the target analyte using measurement signals from at least two of the plural sensing sites.

Embodiments provide systems and methods which have improved sensor functionality and versatility, including a wider sensor dynamic sensing range without sacrificing accuracy, improved speed of analysis and improved confidence in the identification of a target analyte.

The sensor assembly includes plural sensing sites, each having a different capture species which specifically bind to, but have a different binding affinity with, the same target analyte. Binding affinity defines the nature of the interaction between two entities which non-covalently bind together, in this case the respective capture species and target analyte, and can be represented as the binding constant ($K_a$) or the inverse of the binding constant, the dissociation constant ($K_D$). The presence of plural sensing sites, each having a different capture species, enables the abovementioned advantages. For example, the presence of two different, individually-readable sites with a specificity for the same target analyte allows for each site to have a different dynamic sensing range based at least in part on the affinity of the respective capture species. Depending on the property of the target analyte in the sample, the optimal site for determining the property can be used. This could be a selection of a sensing site in which the amount of target analyte is expected to be within a substantially linear or well-defined range. For example, this could be the optimal site for determination of a particular concentration. The system can therefore alter detection properties such as the sensitivity or detection limits depending on the property of the target analyte. Embodiments may use a site with a broad response to estimate the property of the analyte, before a site with an optimal configuration is selected for the measurement.

The use of capture species of different affinities can also enable faster measurements. Embodiments may use a site with a fast response to estimate the property of the analyte, before a site with an optimal configuration is selected for the measurement. As set out in more detail below, typically a capture species with a higher Kowill provide a faster response but this requires compromises on other factors, and so it may be advantageous to use such a site for an initial measurement. In other or additional embodiments, a sensing site where the expected concentration of the target analyte is well above the lower limit of detection (LOD) may be used so that the expected concentration can be projected/estimated quickly, before the optimal site is used for a full measurement, based on this estimate/projection.

Moreover, the use of plural sites together can improve the accuracy of the determination. Each of the sites will have a different response to the presence of the analyte and this response can be combined to provide a more accurate determination. Each may provide a response which together can be used to observe a fingerprint or response across the plural sites which is unique to the presence and amount of the target analyte. This may be based on the binding profile and/or a kinetic analysis of the measurement signal for each site. Each may also have an identical or near identical baseline in the absence of the analyte, which can be used to compensate for noise and increase accuracy.

The use of capture species of different affinities is advantageous compared to using different configurations of the same capture species (e.g. different densities and/or sensing site sizes) because of the different responses together these can be used as a fingerprint, as compared to using different configurations of the same capture species (e.g. different densities and/or sensing site sizes). This also provides added confirmation over sensor assemblies based on a single capture species.

Determination of the property

The methods, systems and sensor assemblies disclosed herein can be used to determine or measure a property of an analyte in a sample, such as an analyte characteristic. In certain embodiments, this can be selected from the concentration of the analyte in the sample, the diffusion constant of the analyte (e.g. rate of diffusion measured in $m^2/s$) in the sample, or a combination thereof. The terms "analyte concentration" or "concentration of the analyte" as used herein may, in certain embodiments, refer to the activity of the analyte. The activity of the analyte may provide a measure of the effective concentration of the analyte in a sample matrix.

The analyte may, for example, be selected from a molecular species, a metal ion, a virus, and a microorganism. Biomolecule analytes are particularly useful and may, for instance, be a hormone selected from an eicosanoid, a steroid, an amino acid, amine, peptide or protein.

In embodiments, determination of the property comprises using measurement signals from at least two of the plural sensing sites. That is, at least two of the sensing sites are used to arrive at the determination. This may be directly by using the measurement signals in the calculation of the property (i.e. that the determination is based (at least in part) on the measurement signals from at least two of the sensing sites) or it may be that the measurement signal of at least one sensing site is used to determine which other sensing site will provide the optimal measurement, before the measurement signal from the other sensing site is used in the calculation of the property (only, or together with other sensing site(s)).

*Sensor fusion*

Accordingly, in one embodiment, the control unit being configured to use measurement signals from at least two of the sensing sites in determining the property of the analyte comprises: determining a property of the target analyte based on measurement signals from at least two of the plural sensing sites. In an embodiment of the method, determining the property of the analyte using measurement signals from at least two of the plural sensing sites comprises determining a property of the target analyte based on measurement signals from at least two of the plural sensing sites. That is, a measurement signal from at least two different sensing sites is used in the calculation of the property (e.g. concentration) of the target analyte. This sensor fusion increases the accuracy of the measurement, as detailed above.

*Correction/Compensation*

In one additional or alternative embodiment, the control unit is configured to determine a composite baseline signal based on the measurement signal from at least two of the plural sensing sites in the absence of target analyte on the respective sensing sites; and the control unit is configured to determine a property of the target analyte based on measurement signals from at least one of the plural sensing sites and the composite baseline signal. In one additional or alternative embodiment of the method, determining a composite baseline signal based on the measurement signal from at least two of the plural sensing sites in the absence of target analyte on the respective sensing sites; and determining a property of the target analyte based on measurement signals from at least one of the plural sensing sites and the composite baseline signal.

In such embodiments, a baseline signal from at least two of the plural sensing sites can be obtained and combined during the step of determining the property so as to correct or compensate for e.g. baseline noise. This can be used in lieu of or in addition to a control. The composite signal aggregates or combines the signal. This can, in embodiments, comprise measurement of a baseline signal on a first and a second of the plural sensing sites, and comprise determining the property based on a measurement signal from at least one of the first, second sites and/or another of the plural sites (where more than two are present). Thus, the method and system need only have a measurement signal from one sensing site for the determination, but also use measurements from at least one additional sensing site to more accurately determine the property.

*Optimisation of a site*

In one embodiment, the control unit being configured to determine the property of the analyte comprises: determining an initial property measurement based on the measurement signal from at least a first sensing site of the plural sensing sites; selecting at least one other sensing site of the plural sensing sites for measurement of the property, based on the initial property measurement; and determining the property of the target analyte based on the measurement signal from the selected at least one other sensing site. In one embodiment of the method, determining the property of the analyte comprises: determining an initial property measurement based on the measurement signal from at least a first sensing site of the plural sensing sites; selecting at least one other sensing site of the plural sensing sites for measurement of the property, based on the initial property measurement; and determining the property of the target analyte based on the measurement signal from the selected at least one other sensing site.

This advantageously can speed up measurement process, provide improved sensitivity and increase the accuracy of the method over existing sensing systems. The initial property measurement can provide an initial indication of the property of the analyte (e.g. the concentration or presence), and this can then be used to determine the optimal sensing site(s) for measurement of the property.

For example, this may enable a sample having an unknown concentration of target analyte to be provided to the sensor assembly. The system may make an initial determination (such as a projection or estimate) of the concentration based on the measurement signal from one or several sites. The sensor assembly can then be reconfigured based on this initial determination or estimate of the concentration to ensure that sample is analysed by the sensing site with a particular (or optimal) operating range of the sensor assembly. For example, this might be an optimised concentration range within which the accuracy of the measurement is higher or may be used to increase the sensitivity of the measurements. This flexibility can allow the sensor assembly to be used with varying concentration ranges without compromising the accuracy of the measurements, for example.

By initial property measurement (or initial indication), it is meant that an initial measurement (determined or estimated) of the property (e.g. concentration). In some embodiments, the initial indication may be less accurate or rely on less information than a normal measurement. For example, it may be that the measurement time for the initial determination is quicker than a standard measurement time for a sample. This may be where the concentration is estimated or projected. Alternatively, it may be that the initial measurement is identical to a normal measurement and that it only differs in that the non-initial measurement is carried out under optimal sensor configurations. That is, the initial measurement may use a first sensing site whereas a measurement on which the property determination is based is carried out once the sensor assembly has been configured based on the initial indication (e.g. after the initial measurement has been used to determine the optimal or preferred sensing site).

In one embodiment, the sample may be provided to each of the plural sensing sites and the initial property measurement may be used to determine from which of the sensing sites the measurement signal(s) obtained should be used in the determination of the property. This has the advantage of being quick, as the binding of the target analyte to the capture species on the optimal site (as well as the other sites) is already underway as the initial property measurement is determined. The selection of the sensing site(s) used in the determination is then carried out as a signal processing step (e.g. by the control unit). In other embodiments, the sample may only be provided to the sites from which the measurement signal is used in determination of the initial property measurement, and subsequently sample may be provided to the sites which are to be used in the determination of the property. This may be by a fluid distribution apparatus provided to the system, and may be under the control of the control unit.

Selection of the sensing site(s) for determination of the property based on the initial property measurement can be based on a comparison of the initial property measurement with at least one parameter of the sensing sites. In one embodiment, each of the plural sensing sites has a corresponding limit of detection (LOD) and dynamic range for measuring the property of the target analyte, determined at least in part by the binding affinity ($K_A$) of the respective capture species; and wherein selecting at least one other sensing site of the plural sensing sites for measurement of the property is based on a comparison of the initial property measurement with the LOD and/or dynamic range of the other of the plural sensing sites. In embodiments of the method, each of the plural sensing sites has a corresponding limit of detection (LOD) and dynamic range for measuring the property of the target analyte, determined at least in part by the binding affinity ($K_A$) of the respective capture species; and selecting at least one other sensing site of the plural sensing sites for measurement of the property is based on a comparison of the initial property measurement with the LOD and/or dynamic range of the other of the plural sensing sites. LOD as used herein refers to the lower LOD. The LOD may be determined by the point at which the signal-to-noise ratio is 20dB (i.e. a ratio of 20). The upper LOD may be determined by the saturation point of the sensing site.

For example, the sensor may comprise a first sensing site configured to provide a linear or substantially linear response in a first concentration range of the target analyte and a second sensing site of the plural sensing sites is configured to provide a linear or substantially linear response in a second concentration range of the target analyte, the second concentration range being different to the first concentration range. The control unit is configured to determine the initial property measurement of the target analyte based on a first measurement signal from the first sensing site and subsequently determine whether to use the first sensing site or second sensing site for the target analyte based on the initial property measurement and a comparison of the first concentration range and the second concentration with the initial concentration measurement. This can be a determination whether the initial concentration range falls within either of the first concentration range and the second concentration range, or to which is it the closest (e.g. closest to the mid-point).

This may be a comparison of absolute values or relative values of the sensing sites. For example, it may be that sites are designated as "high" or "low" concentration relative to one another, and a predetermined threshold is used for each. In other examples, each may have a predetermined or determined LOD and dynamic range and there may be a direct comparison of the initial property determination to determine which of these sensing sites is closest to or encompasses the value determined. Where there are plural sites that could be used, this may be determined by the mid-points of ranges, or based on other factors, such as breadth of dynamic range or speed of measurement.

By "dynamic range", it is meant the property range (e.g. concentration range) across which the sensing site can provide a measurable signal. For example, from the lower measurable output (also known as the LOD) to the highest measurable output (which can be set at a particular point for each range, either based on saturation limit or it may be at a predetermined change in response). Each of the plural sensing sites will have a limit of detection (LOD) and dynamic range for measuring the property of the target analyte. In some embodiments, where quantitative measurement is required and the limit of quantitation (LOQ) is different to the LOD, then the range used may be from the LOQ to an upper LOQ. This range is interchangeable with dynamic range herein, such that embodiments may utilise either.

The dynamic range is determined at least in part by the binding affinity of the respective capture species. Affinity (as defined by Ka or $K_D$) of the target analyte to the capture species can contribute to the sensitivity of the sensing site and thus dynamic range and LOD/LOQ. Given that sensing conditions (e.g. concentration of the analyte in the sample, pH, temperature) will be identical or nearly across the sensing sites, the affinity will provide a large degree of influence on the LOD and dynamic range.

It will be appreciated that other factors will also influence the dynamic range, such as amount of the capture species provided on the surface and the size of the sensing site. As noted above, the sensing sites are configured so that the response to the target analyte is different at least in part based on the different in the affinities of the capture species (to the target analyte). That is, there may be contributions from other factors in addition, but the affinity contributes to this different in response. In some embodiments, this different in response may be based entirely or substantially entirely on the difference in affinities. For example, in some embodiments, at least two of the plural sensing sites may be the same size or substantially the same size with the same or substantially the same amount of capture species thereon (defined by the density of the capture species on the sensing site and/or the number of sites available for binding the target analyte/unit size). This may be for all of the plural sensing sites. In some embodiments, the different in size and/or density between sites may be less than 10%, such as less than 1%. In some embodiments, the difference in affinities leads to a difference in the shape of the signal plotted overtime. This can provide useful information, even where the LOD and dynamic range are substantially similar for different sites.

In one embodiment of the determination of the initial property measurement, the first sensing site has a first dynamic range extending from a lower concentration to an upper concentration, and wherein the respective dynamic range of each of other of the plural sensing site(s) extend from respective lower concentrations and upper concentrations falling between the lower concentration to an upper concentration of the first dynamic range. In other words, the first dynamic range encompasses the dynamic range(s) of the other of the plural sensing site(s). Where there are other plural sensing sites (e.g. the plural sensing sites comprises at least three sensing sites, including the first sensing site), the first sensing site has a dynamic range which is broader than (i.e. encompasses) the corresponding dynamic range of all of the other of the plural sensing sites. In this way, the first sensing site can be used to provide an initial property measurement across a broad range of concentrations. This initial property measurement can then be used to determine which of the smaller-range other sensing sites, which may provide a higher level of accuracy or a faster response time than the broad range first sensing site, should be used for the determining of the property.

In one embodiment, the first sensing site has a dynamic range having a mid-point $M_1$, and the sensor assembly comprises at least a second sensing site (of the plural sensing sites) having a dynamic range with a mid-point $M_2$ and at least a third sensing site (of the plural sensing sites) having a dynamic range with a mid-point $M_3$, and wherein $M_2 > M_1 > M_3$. In such an embodiment, the sensor assembly thus plural sensing sites in the form of at least three (first, second and third) sensing sites. Each site x has a dynamic range with a midpoint $M_x$, (between the lower limit and the upper limit) where the first sensing site (i.e. the site which is used to determine the initial property measurement) has a midpoint $M_1$ between the mid-point of the second sensing site $M_2$ and that of the third sensing site $M_3$. Typically a dynamic range is more precise in the region at or around the midpoint of the dynamic range where the response of the sensing site tends to be linear or more linear than at the end points. This allows for a range of sensing sites, which in turn can increase the overall dynamic range and performance of the system. In some embodiments, $M_2$ and $M_3$ outside the dynamic range of the first sensing site (i.e. $M_2$ is less than the LOD of the dynamic range of the first sensing site ($LOD_1$). In some embodiments, $M_2$ is less than or equal to $0.25*(M_1-LOD_1)$ and $M_3$ is greater than or equal to $0.75*(M_1-LOD_1)$. In some embodiments, $M_2$ is in the range of from $LOD_1$ to less than $0.5*(M_1-LOD_1)$ and $M_3$ is in the range of from $0.5*(M_1-LOD_1)$ to the upper limit of the dynamic range of the first sensing site. In some embodiments, $M_2$ is in the range of from $LOD_1$ to less than $0.25*(M_1-LOD_1)$ and $M_3$ is in the range of from $0.75*(M_1-LOD_1)$ to the upper limit of the dynamic range of the first sensing site.

In some embodiments, the control unit is configured to determine the property of the target analyte based on the rate of change in a measurement signal of at least one of the plural sensing sites. In some embodiments, the method may comprise determining the property of the target analyte is based on a rate of change in a measurement signal of at least one of the plural sensing sites. These may be based on at least two of the plural sensing, in some embodiments all of the plural sensing sites. Rate of change can help to build up the picture or fingerprint providing a higher degree of accuracy and certainty. Unlike typical electrochemical measurements, the rate of change is particularly beneficial in these methods given the use of several sites interacting with the same target analyte but likely providing a varying response.

Capture species

As set out herein, each of capture species has a different binding affinity. Binding affinity can be defined by the binding constant $K_A$ or the inverse of $K_A$, the dissociation constant $K_D$. $K_A$ and $K_D$ are equilibrium constants relating to the rate of binding of the target analyte to the capture species (rate constant $k_{on}$) and the subsequent rate of dissociation of the target analyte from the capture species (rate constant $k_{off}$) (or a detection species and the target analyte). In particular:

$$K_A = \frac{k_{on}}{k_{off}} \qquad K_D = \frac{k_{off}}{k_{on}} = \frac{1}{K_A}$$

$K_D$ is in molar concentration (M or mole/L), $K_A$ is $M^{-1}$ (or L/mole), $k_{off}$ and $k_{on}$ are moles/second.

Affinity is an important parameter, since it characterises the relationship between the respective capture species and the target analyte. It has a significant influence on speed of the measurement as well as sensitivity and dynamic range. Moreover, by providing different capture species with different affinities, different sensing sites will have different parameters, such as speed of binding ($k_{on}$), sensitivity and/or dynamic range and thus provide a different response to the same target analyte. For example, a higher $K_A$ (or lower $K_D$) will provide a higher sensitivity and lower detection limits.

One of the major rate determining steps in measurement using a target analyte and a capture species is the reaction speed. The speed (rate) will depend on a number of factors, including the number (or density) of capture species, the number of already bound capture species, the size of the sensing area, and importantly, the rate of binding and dissociation (i.e. characterized by $K_A$ or $K_D$). In equilibrium, this can be written as:

$$N = b_m A \frac{f}{1+f}, \quad f = \frac{c}{K_D}$$

where N is number of bound capture species, $b_m$ is surface density of capture species (number/cm$^2$), A is sensing area (cm$^2$) and c is concentration (M).

The time constant $\tau$ for the sensor is determined by:

$$\tau = \frac{1}{k_{off} + c\, k_{on}}$$

At low concentrations, for example, this is in essence simplified to $1/k_{off}$. A fast response at these concentrations would therefore require a large $k_{off}$, which competes with small $K_D$ or a high $K_A$. As such, there is typically a trade-off when selecting a suitable capture species which had as a high affinity but without sacrificing measurement speed. In embodiments, the sensing sites can be provided with different affinities such that this compromise need not be made.

The affinity (defined by affinity constant $K_A$ or the dissociation constant $K_D$) can be measured by any known method, provided that the method used for each capture species is the same or comparable to determine the relative affinities of the capture species.

In embodiments, the capture species of the first sensing site has a rate of binding to the target analyte ($k_{on}$) which is higher than the rate of binding to the target analyte ($k_{on}$) for the capture species of the other of the plural sensing sites; and/or wherein the capture species of the first sensing site has a binding affinity ($K_a$) which is less than the binding affinity ($K_a$) of the capture species of the other of the plural sensing sites. Such a site can be used to provide a quicker measurement and thus provide an initial indication of the approximate property, at which point the most appropriate sensing site (and capture species) can be selected for use of the measurement signal.

In some embodiments, each capture species provided on each of the sensing sites has an affinity defined by $K_D$ of from $1 \times 10^{-4}$ to $1 \times 10^{-15}$ M (i.e. micromolar ($\mu$M) to femtomolar (fM). This may be from $1 \times 10^{-7}$ to $1 \times 10^{-15}$ M or from $1 \times 10^{-4}$ to $1 \times 10^{-12}$ M (i.e. micromolar ($\mu$M) to picomolar (pM)). In some embodiments, each of the capture species provided on each respective sensing site is a capture antibody and each capture antibody has a dissociation constant ($K_D$) of from $10^{-4}$ to $10^{-15}$. A capture antibody is an antibody capture species. In some embodiments, this may be from 10 pM to 10 nM or from 1 nM to 10 pM. Typical $k_{on}$ and $k_{off}$ values in embodiments at these $K_D$ values range from $1e^4$ to $1e^6$/M/s and $1e_{-3}$ to $1e^{-6}$/s, respectively. For example, in one embodiment, the capture species of each of the plurality of sites may have an affinity which is at least one order of magnitude different (x10) to the other capture species provide on the other of the plurality of sites. In some embodiments, there may be at least first and second sensing sites, the first having a first capture species with a $K_D$ of from $1 \times 10^{-4}$ to $1 \times 10^{-12}$ M and the second having a second capture species with a $K_D$ of from $1 \times 10^{-13}$ to $1 \times 10^{-15}$ M. In some embodiments, there may be at least first and second sensing sites, the first having a first capture species with a $K_D$ of from $1 \times 10^{-4}$ to $1 \times 10^{-9}$ M and the second having a second capture species with a $K_D$ of from $1 \times 10^{-10}$ to $1 \times 10^{-15}$ M. In other embodiments, the difference of the $K_D$ of the capture species on each site may be less but still different so that the difference has an effect on the response.

It will be appreciated that affinity can vary for a number of reasons. Identification of capture species of different affinities can be achieved through existing methods known in the art, including antibody and antigen screening.

In embodiments, binding affinity ($K_A$ and/or $K_D$), $k_{on}$ and/or $k_{off}$ can each be measured by one of surface plasma resonance (SPR), biolayer interferometry (BLI) and ELISA. In embodiments, binding affinity ($K_A$ and/or $K_D$), $k_{on}$ and/or $k_{off}$ can each be measured as set out in "How to measure and evaluate binding affinities" Jarmoskaitelshraq et Al. 2020 eLife 9:e57264 https://doi.org/10.7554/eLife.57264, which is incorporated herein by reference. In particular, in some embodiments, the binding affinity ($K_A$ and/or $K_D$), $k_{on}$ and/or $k_{off}$ can be measured in accordance with the "kinetic approach" method set out in Appendix 1 of "How to measure and evaluate binding affinities". In some embodiments, this can include measurements at 25 °C and/or 0°C. In some embodiments, $k_{on}$ is measured as set out in this method "$k_{on}$ chase" in Appendix 1. In some embodiments, these values (binding affinity ($K_A$ and/or $K_D$), $k_{on}$ and/or $k_{off}$) are measured across concentrations of 0.05nM to 0.30nM in 0.05nM increments.

In other embodiments, binding affinity ($K_A$ and/or $K_D$), $k_{on}$ and/or $k_{off}$ can each be measured using Biolayer Interferometry (BLI), for example using ForteBio Octet RED384 instrument and, in some embodiments, the method set out in "Off-rate screening for selection of high-affinity anti-drug antibodies" Ylera et. Al Analytical Biochemistry, Vol 441, Issue 2, 2013, Pages 208-213, ISSN 0003-2697, which is incorporated herein by reference.

Analyte/Capture Species

The capture species on each sensing site specifically binds to the target analyte. By this it is meant that each of the capture species binds to a particular site or moiety on the target analyte. There may be plural interactions between the capture species and target analyte. Each capture species may bind to a different site of moiety on the target analyte, which may provide the different affinities.

Any suitable analyte capture species can be selected according to the target analyte which is intended to be sensed by the sensor assembly. For example, each capture species may comprise an antibody with specificity for a particular antigen. In such an example, the analyte may take the form of the antigen. More generally, each capture species may, in some embodiments, comprise at least one selected from a protein, a peptide, a carbohydrate, and a nucleic acid. The protein may, for example, be an enzyme, such as an enzyme having specificity for the analyte. In other non-limiting examples, the protein is an antibody. In the latter case, the analyte may be an antigen which is specifically bound by the antibody. Each capture species may, for instance, comprise or be defined by an antigen. In this case, the analyte may be a species, such as an antibody, which is specifically bound by the antigenic capture species. The antigen may be or comprise, for example, a protein, a peptide, a carbohydrate, such as a polysaccharide or glycan. In an embodiment, each capture species is a capture antibody.

In an embodiment, each capture species comprises an aptamer. An aptamer may be defined as an oligonucleotide or peptide configured to bind the analyte. Such an aptamer may, for example, be configured to interact with, for example bind, various analyte types, such as small molecules, for example amino acids or amines, proteins, metal ions, and microorganisms.

One common assay type is enzyme-linked immunosorbent assay (ELISA). ELISA is a so-called sandwich-assay used for quantifying analytes such as peptides, proteins, antibodies, and hormones. These use a recognition element for selectively (specifically) interacting with, for example binding, the analyte of interest. The recognition element is immobilized on a suitable support. For example, an antigen is immobilized on the support and then complexed with an antibody that is linked to an enzyme. In one embodiment, the method further comprises providing a detection species to the sample matrix, the detection species specifically-binding to the target analyte. The detection species may be provided when the analyte is specifically bound to the capture species or before this occurs. The detection species may be an amplification species or may be a different species, for example provided with the purpose of increasing the selectivity of the measurement. This can be a sandwich assay. In some embodiments, the method is a sandwich ELISA method of detecting a property of an analyte, with each capture species being a capture antibody, the detection species being a detection antibody which may comprise double stranded DNA provided thereon. The DNA may be bound to the detection antibody via a linker, such as streptavidin and biotin.

Sensor assembly

The sensor (or sensing) assembly comprises plural sensing sites, each of the sensing sites comprising a capture species configured to specifically bind with the target analyte and wherein each sensing site provides a measurement signal indicative of the interaction of the respective capture species with the target analyte. In other words, the sensor assembly comprises: a first sensing site comprising a first capture species configured to specifically bind with analyte provided on the sensing site, the first capture species having a first binding affinity for the analyte and the first sensing site providing a first measurement signal indicative of the interaction of the first sensing site (i.e. the first capture species) with the analyte; and a second sensing site comprising second capture species configured to specifically bind with analyte provided on the sensing site, the second capture species has a second binding affinity for the analyte and the second sensing site providing a second measurement signal indicative of the interaction of the second sensing site (i.e. the second capture species) with the analyte, wherein the first binding affinity and the second binding affinity are different. The first and second sensing sites accordingly provide the plural sensing sites. In some embodiments, the sensor assembly may further comprise a third sensing site comprising a third capture species having a third binding affinity for the analyte and the third sensing site providing a third measurement signal indicative of the interaction of the third sensing site with the analyte, wherein the third binding affinity is different to the first and second binding affinities.

In embodiments, each sensing site may comprise analyte interaction portions defined by the respective capture species, where each capture species is provided adjacent or on at least one surface of the respective sensing site. In some embodiments, each sensing site is functionalized with the capture species. Such functionalization can be achieved in any suitable manner, such as by covalently or non-covalently immobilizing the capture species to the surface. For example, thiol-terminated capture species, such as a thiol-terminated aptamer, can be immobilized, for example grafted, onto the surface of a noble metal, for example gold, electrode.

More generally, the sensing sites are arranged to receive a sample. The sample comprises a sample matrix in which there may be the target analyte and other components. Thus, the sample may comprise a carrier (such as a liquid) and the analyte. The sample matrix may be, for example, blood, urine, sweat, tears, etc., and may (potentially) contain the analyte.

Each sensing site is an individually readable or addressable sensing site so that a measurement signal for each individual site can be obtained. Each sensing site may therefore be provided on a separate sensing element or layer, such as an electrode or field effect transducer (FET). In other embodiments, the sites may be on a common element, with individually addressable regions.

In the methods and system disclosed herein, the transducer mechanism(s) used to determine the measurement signal may be any suitable method of transduction. For example, this may comprise measuring the potential (e.g. voltage), current, permittivity, charge and/or frequency. In some embodiments, each sensing site comprises at least one working electrode (e.g. either forming the sensing surface or adjacent and addressing an element forming the sensing surface). Changes in or interactions with the functional layer formed by the capture species on the surface can be detected through changes in potential. In other embodiments, changes may be determined by monitoring changes in current passing through each sensing site (at a constant potential).

The sensing site can be a surface of a sensing layer (e.g. an electrode). In some embodiments, this may comprise or be formed from copper, nickel, platinum, silver, silver chloride, gold or other noble metals. In some embodiments, this may comprise or be formed from TiOz or indium tin oxide (ITO). Other sensing sites may include a substrate with a coating on which the anchor species is immobilized. For example, the sensing site may be a glass substrate with an ITO coating thereon. In other embodiments, the sensing site may comprise or be formed of carbon (graphene, graphene oxide, or nanotubes), silicon dioxide, aluminum oxide, and/or silicon. Sensing layers can provide immobilization of capture species through both covalent-like interactions (e.g. chemisorption of anchor species onto the surface through chemical bond formation) and non-covalent-like interactions (e.g. physisorption of capture species onto the surface through weaker, often van der Waals, interactions) depending on the identity of the surface and the capture species. Provision of the capture species to the sensing site can be achieved through techniques such as spin-coating, physical vapour deposition or electrophoretic deposition. Alternatively, other methods can include immersion of the sensing site in solution.

## System

The system may be configured to perform any of the method steps disclosed herein. Moreover, any of the embodiments set out herein with respect to the method apply equally to the system, and any of the embodiments set out herein with respect to the system apply equally to the method. In embodiments, the control unit of the system may be configured to carry out any of the method steps set out herein.

In one embodiment, the system may further comprise a signal processing unit configured to process measurement signals received from the sensor assembly; and a property determination unit may receive processed signals and determined the property based on the processed signals. The property determination unit may, in certain embodiments, be configured to determine the property based on (at least) the absolute change in measurement signal and/or the rate of change of the signals. The control unit, property determination unit and/or signal processing unit may each (individually or combined) be a processor or controller. The control unit may incorporate the property determination unit and/or the signal processing unit or may be in addition to one or both of these. The control unit, signal processing unit, and the property determination unit may be implemented in any suitable manner, with software and/or hardware, to perform the various functions required. One or all of the units may, for example, employ one or more microprocessors programmed using software (for example, microcode) to perform the required functions. Examples of processor components that may be employed in various embodiments include, but are not limited to, conventional microprocessors, application specific integrated circuits (ASICs), and field-programmable gate arrays (FPGAs).

In various implementations, the control unit, the signal processing unit, and/or property determination unit may be associated with one or more non-transitory storage media such as volatile and non-volatile computer memory such as RAM, PROM, EPROM, and EEPROM. The non-transitory storage media may be encoded with one or more programs that, when executed on one or more processors and/or controllers, perform the required functions. Various storage media may be fixed within a processor or controller or may be transportable, such that the one or more programs stored thereon can be loaded into the signal processing unit, property determination unit and/or control unit.

In some non-limiting examples, the system includes a user interface, such as a display, for communicating the analyte property determined by the property determination unit. Alternatively or additionally, the system may include a communications interface device, such as a wireless transmitter, configured to transmit the analyte concentration determined by the property determination unit to an external device, such as a personal computer, tablet, smartphone, remote server, etc.

In one aspect, there is provided a computer program comprising computer program code which is configured, when said computer program is run on one or more physical computing devices, to cause said one or more physical computing devices to implement the methods disclosed herein.

In one aspect, there is provided one or more non-transitory computer readable media having a computer program stored thereon, the computer program comprising computer program code which is configured, when said computer program is run on one or more physical computing devices, to cause said one or more physical computing devices to implement the methods disclosed herein.

## Other Implementations

In another aspect, there is provided a system for determining a property of target analyte in a sample, the system comprising: a sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity ($K_a$) for the target analyte, the binding affinity ($K_a$) of each of the capture species being different to the other of the capture species. The system further comprises a control unit configured to determine the property of the target analyte by: determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

In another aspect, a method for determining a property of target analyte in a sample comprises providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity ($K_a$) for the target analyte, the binding affinity ($K_a$) of each of the capture species being different to the other of the capture species. The method further comprises determining the property of the target analyte by: determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

In some embodiments, embodiments of these aspects comprise the same features as the abovementioned embodiments of the earlier aspects. For example, but not limited to, the embodiments in respect of the capture species, the relative properties of the sensing sites (dynamic range, LOD) and the structure of the system and method.

However, in addition, in embodiments these aspects may comprise the use of only the measurement signal from a single (first) sensing site, despite there being plural sensing sites. For example, the sensing site used during the step of determining initial property measurement (i.e. providing the measurement signal) may be the most appropriate site for measurement of the measurement used in determining the property of the target analyte. In such a case, the selection may be of the first sensing site. The initial measurement may be based on a measurement signal obtained over a shorter duration than the duration of the measurement signal used in the subsequent step of determining the property.

In one further aspect, there is provided a sensor assembly for a target analyte in a sample, comprising: plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity ($K_a$) for the target analyte, the binding affinity ($K_a$) of each of the capture species being different to the other of the capture species. In one embodiment, the plural sensing sites comprise: a first sensing site comprising a first capture species, the first capture species having a first binding affinity for the target analyte and the first sensing site providing a first measurement signal indicative of the interaction of the first capture species with the analyte; a second sensing site comprising a second capture species having a second binding affinity for the analyte and the second sensing site providing a second measurement signal indicative of the interaction of the second capture species with the analyte; and a third sensing site comprising a third capture species having a third binding affinity for the analyte and the third sensing site providing a third measurement signal indicative of the interaction of the third capture species with the analyte.

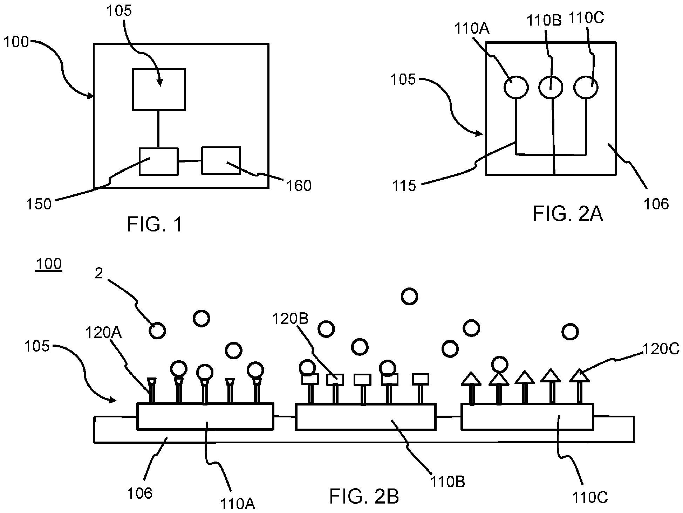

Fig. 1 schematically depicts a system 100 for determining the concentration of a target analyte 2 according to an embodiment and Figs. 2A and 2B schematically depict an embodiment of a sensor assembly 105 used in the system 100 of Fig. 1. Fig. 2A shows a schematic plan view of the sensor assembly 105, which comprises a substrate 106, a first sensing site 110A, a second sensing site 110B, a third sensing site 110C and a track 115. Fig. 2B shows a schematic cross-sectional view of the sensor assembly 105 through a plane dissecting the first sensing site 110A, second sensing site 110B and third sensing site 110C.

Specifically, the system 100 comprises the sensor assembly 105, a signal processing unit 150 and a processor which acts as the control unit 160 and which is used to determine the concentration of the target analyte 2 in the sample.

Specifically, the sensor assembly 105 comprises plural sensing sites in the form of the first sensing site 110A, second sensing site 110B and third sensing site 110C. Each of the sensing sites 110A-110C are formed of an individually addressable electrode functionalised with a capture species. The first sensing site 110A comprises a first capture species 120A, the first capture species 120A having a first binding affinity for the target analyte 2, and provides a first measurement signal indicative of the interaction of the first sensing site 110A (i.e. the first capture species 120A) with the target analyte 2. The second sensing site 110B comprises a second capture species 120B having a second binding affinity for the target analyte 2 and provides a second measurement signal indicative of the interaction of the second sensing site 110B with the target analyte 2. The third sensing site 110C comprises a third capture species 120C having a third binding affinity for the target analyte 2 and provides a third measurement signal indicative of the interaction of the third sensing site 110C with the target analyte 2. Each of the first, second and third capture species 120A-120C on each of the first, second and third sensing sites 110A-110C is different to the capture species of the other sites, with each of the first, second and third binding affinities for the target analyte being different. Each of the first, second and third sensing sites 110A-110C are electrically connected to the signal processing unit 150 and control unit 160 by track 115 so that the respective first, second or third measurement signal can be obtained by the signal processing unit 150.

The control unit 160 is configured to use at least two of the first, second and third measurement signals from at least two of the first, second and third sensing sites 110A-110C. Two different methods of using the system 100 of Fig 1 and the sensor assembly 105 of Figs. 1A and 1B will now be described.

In use, one embodiment of the use of the system 100 comprises providing a sample with an unknown concentration of the target analyte 2 to the sensor assembly 105. In this embodiment, the sample may be provided to each of the first, second and third sensing sites 110A-110C simultaneously. As the target analyte 2 reaches the surface of each of the first, second and third sensing sites 110A-110C, it will interact with the first, second and third capture species 120A-120C, respectively. However, in view of the different affinities of each of the first, second and third capture species 120A-120C, the speed and nature of the binding of the target analyte to the capture species of each will be different and hence each of the first, second and third sensing sites 110A-110C will produce a different response in the presence of the analyte.

The control unit 160 is configured to cause the signal processing unit 150 to interrogate the first, second and third sensing sites 110A-110C providing first, second and third measurement signals, respectively. Each of the signals is indicative of the binding and make be taken over a predetermined time period, until it is deemed that a sufficient response has been obtained (indicative of a sufficient degree of binding) or until equilibrium is reached.

In this embodiment, the control unit 160 determines the concentration of the target analyte 2 in the sample by using the first, second and third measurement signals from the first, second and third sensing sites 110A-110C, respectively. As each provides a different measurement signal, the fusion of the three signals increases the accuracy of the measurement. For example, each of the first, second and third measurement signals will provide a different response which together will create a unique signature for the particular analyte concentration. This signature is confirmed or created by the use of plural measurement signals. Moreover, the measurement signal from at least two of the first, second and third sensing sites 110A-110C in the absence of binding between the target analyte and the respective first, second or third capture species 120A-120C can be used to generate a measurement of the background and be used in a compensation step for correcting or adjusting the signal for the background.

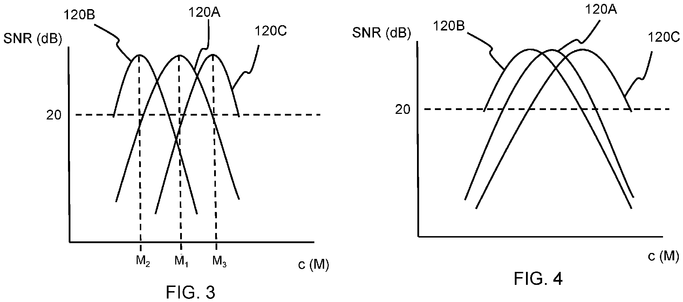

Fig. 3 provides a graph depicting SNR vs concentration for three different capture species with different $K_D$ values for a particular target analyte. Specifically, there are three curves showing SNR (dB) vs concentration (M) for the first capture species 120A (middle curve), the second capture species 120B (left hand curve) and the third capture species 120C (right hand curve), in one embodiment. In this embodiment, the second capture species 120B has a $K_D$ of 10 fM , the first capture species 120A has a $K_D$ of 1 pM and the third capture species 120C has a $K_D$ of 1 nM. The first, second and third sensing sites 110A-110C are the same size and have the same density of respective first, second and third capture species 120A-120C as each other.

As can be seen from Fig. 3, the different affinities (as defined by $K_D$) result in different curves across the concentration range. Specifically, SNR varies across the concentration range for each curve, but each curve has a different range across which it varies. This means that each has a different upper LOD, lower LOD and dynamic range (between the upper LOD and lower LOD). Accordingly, each of the first, second and third sensing sites 110A-110C have a different dynamic range and different upper and lower limits. In this case, the first sensing site 110A has a dynamic range with a midpoint $M_1$ indicated by the peak of the curve, the second sensing site 110B has a dynamic range with a midpoint $M_2$ and the third sensing site 110C has a dynamic range with a midpoint $M_3$.

This means that the responses across each of the sensing sites 110A-110C will be different, and some may be more accurate than others at particular concentration ranges. In Fig. 3, the upper LOD and lower LOD have been determined assuming a lower acceptable SNR limit of 20 dB (as is a well-established lower SNR limit in sensing systems). The affinities of the first, second and third capture species 120A-120C in this embodiment have been selected such that there is overlap of dynamic ranges between at least two of the sites.

In the context of this method, as the target analyte 2 is provided to the first, second and third sensing sites 110A-110C, the first, second and third measurement signals will provide a response, depending on what the concentration is. In the case of a concentration which falls within a concentration which is within the dynamic ranges of the first sensing site 110A and the second sensing site 110B, the control unit 160 may be configured to determine the concentration using the first and second measurement signals (only). Combining these signals will provide improved accuracy over the use of just one. In some embodiments, the third sensing site 110C could be used as a pseudo-control or a control. Similarly, should the concentration of the target analyte 2 be within the dynamic ranges of the first and third sensing sites 110A, 110C, then the control unit may be configured to determine the concentration using the first and third measurement signals (only).

It will be appreciated that the affinities of the first, second and third capture species 120A-120C, and the configuration of the sensing sites 110A-110C (e.g. density of capture species and size of sensing site) may be selected so to have different degrees of overlap or coverage of an overall sensor assembly dynamic range (i.e. a composite dynamic range provided by the three sensing sites 110A-110C as a whole) than that depicted in Fig. 3. For example, Fig. 4 depicts the same graph as Fig. 3 but for an example where there is significant overlap between the dynamic ranges of the first, second and third sensing sites 110A-110C through the selection of the affinities of the first, second and third capture species 120A-120C. Where the target analyte 2 has a concentration falling within the middle of the dynamic range of the sensor assembly 105 as a whole, the presence of the target analyte 2 will be detected on each of the sensing sites 110A-110C such that the determination of the property can be based on the first, second and third measurement signals together.

Comparing the configurations depicted in Figs. 3 and 4, the configuration provided by Fig. 3 may be advantageous in situations where the target analyte 2 may be found over a greater concentration range in the samples. The configuration provided by Fig. 4 may be advantageous in situations where the target analyte 2 may be found in a narrower concentration range but the accuracy of the measurement is particularly important. Both provide significant advantages over sensor assemblies employing one capture species.

In use, in another embodiment of the system 100, active feedback is used whereby the system 100 is used to obtain an initial measurement of the concentration, and the control unit 160 determines the optimal configuration of the sensor assembly 105 for the concentration detected in the initial measurement.

In particular, the sample is provided to the first sensing site 110A and the control unit 160 determines an initial property measurement based on the first measurement signal from the first sensing site 110A. This can be taken over a predetermined time period, until it is deemed that a sufficient response has been obtained (indicative of a sufficient degree of binding) or can be until equilibrium is reached.

The control unit 160, based on the initial property measurement, selects at least one of the second sensing site 110B and third sensing site 110C to use as the basis for the determination of the concentration. As the second sensing site 110B and third sensing site 110C each have different capture species and corresponding different affinities, the response from each of the second and third sensing sites 110B, 110C will be different and different to the first sensing site 110A. The control unit 160 in this embodiment determines which of the second and third sensing sites 110B, 110C should be used to measure the concentration, which can be based on the dynamic sensing ranges of second and third sensing sites 110B, 110C. The control unit 160 is further configured to determine the concentration of the target analyte 2 in the sample based on the second and/or third measurement signals from the second and/or third sensing sites 110B, 110C. This creates a sensor assembly 105 which is versatile can be optimised on-the-fly to adapt to the specific sample.

In one embodiment of this method, the sample may be provided to each of the first, second and third sensing sites 110A-110C simultaneously, or at least prior to the selection of a measurement signal for the determination of the concentration. In this way, selection of the measurement signal can be carried out as a processing step (e.g. by the control unit) rather than requiring subsequent manipulation of the sensor assembly 105 and/or sample. In other embodiments, the initial property determination may be made prior to obtaining the selected measurement signal. In other words, prior to addressing the second or third sensing site 110B, 110C or even prior to providing the sample to the second and/or third sensing site 110B, 110C (e.g. by a fluidic distribution system or by a user). This can be advantageous in some systems as the provision of this optimization functionality at device level can reduce the reliance on associated electronics to provide improved accuracy and sensitivity (e.g. a dynamic sensing range).

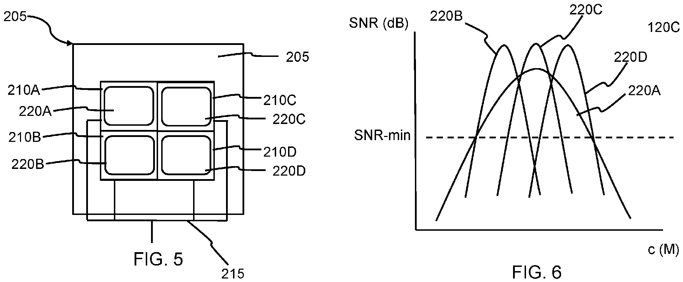

Fig. 5 depicts an embodiment of a sensor assembly 205 which can be used in the system 100 of Fig. 1. The sensor assembly 205 comprises plural sensing sites in the form of a first sensing site 210A, a second sensing site 210B, a third sensing site 210C and a fourth sensing site 210D. Each of the sensing sites 210A-210C are formed of an individually addressable electrode functionalised with a capture species. The first sensing site 210A comprises a first capture species 220A, the first capture species 220A having a first binding affinity for the target analyte 2, and provides a first measurement signal indicative of the interaction of the first sensing site 210A with the target analyte 2. The second sensing site 210B comprises a second capture species 220B having a second binding affinity for the target analyte 2 and provides a second measurement signal indicative of the interaction of the second sensing site 210B with the target analyte 2. The third sensing site 210C comprises a third capture species 220C having a third binding affinity for the target analyte 2 and provides a third measurement signal indicative of the interaction of the third sensing site 210C with the target analyte 2. The fourth sensing site 210D comprises a fourth capture species 220D having a third binding affinity for the target analyte 2 and provides a fourth measurement signal indicative of the interaction of the fourth sensing site 210D with the target analyte 2. Each of the first to fourth capture species 220A-220D on each of the first to fourth sensing sites 210A-210D is different to the capture species of the other of the first to fourth sensing sites 210A-210D, with each of the first to fourth binding affinities for the target analyte 2 being different. Each of the first to fourth sensing sites 210A-210C are electrically connected to the signal processing unit 150 and control unit 160 by track 215 so that the respective first to fourth measurement signals can be obtained by the signal processing unit 150.

Fig. 6 provides a graph depicting SNR vs concentration for first to fourth capture species 220A-to 220D

with different $K_D$ values for a particular target analyte. Specifically, there are four curves showing SNR (dB) vs concentration (M) for each of the first to fourth capture species 220A-220D. In this embodiment, the affinities of the second, third and fourth capture species 220C-220D are such that these provide three similar breadth dynamic ranges but across three different concentration ranges. As can be seen in Fig. 6, the second capture species 220B provides a dynamic range covering a second concentration range in a low concentration range, the third capture species 220C provides a dynamic range covering a third concentration range which partially overlaps with a third concentration range and covers a mid-concentration range and the fourth capture species 220D provides a fourth concentration range in an upper concentration range, but also partially overlaps with the third concentration range. In this embodiment, by virtue of the affinity of the first capture species 220A and the configuration of the sensing surface (e.g. size of the first sensing size 210A and density of the first capture species 220A), the dynamic range for the first sensing site 210A is broad and extends across the whole breadth from the lower LOD of the second concentration range to the upper LOD of the fourth concentration range.

This configuration of the first to fourth sensing sites 210A-210B provides a sensor assembly 205 and system 100 which can be used to determine a property of any of the methods disclosed herein.

In use, the system 100 incorporating the sensor assembly 205 in this embodiment can be used to determine an initial property measurement based on the first measurement signal from the first sensing site. The breath of the first sensing site 210A dynamic range enables an initial concentration to be determined, which may be an estimate or a projection based on the first measurement signal. However, as can be seen in Fig. 6, first sensing site 210A generally has a lower SNR (and hence level of sensitivity and precision), particularly at the lower and upper quartiles of the concentration ranges, than the peaks of the second, third and fourth sensing sites 210B-210C. Accordingly, the control unit 160 in one embodiment selects at least one other sensing site of the second to fourth sensing sites for measurement of the property, based on the initial concentration and a comparison of the dynamic ranges depicted in Fig. 6. This may be only one of the second to fourth sensing sites 210B-210D, for example where the initial concentration indicates that this only falls within the dynamic range of one of the second to fourth sensing sites 210B-210D, or may be plural of the second to fourth sensing sites 210B-210D where the indication suggests that this is or may be within the dynamic range of plural of the second to fourth sensing sites 210B-210D.

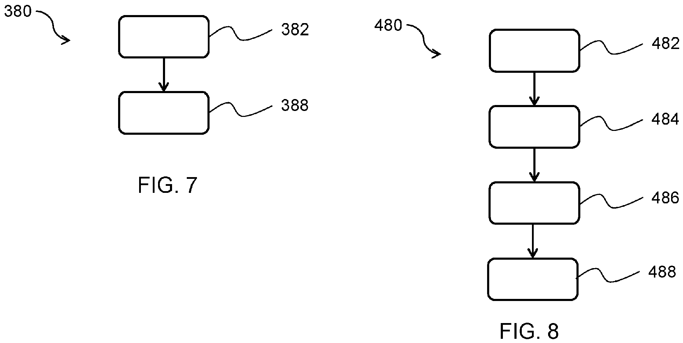

Fig. 7 schematically depicts a method for determining a property of target analyte in a sample 380, according to an embodiment. The method comprises:

First, providing a sensor assembly 382, the sensor assembly comprising plural sensing sites, each of the sensing sites comprising a capture species configured to specifically bind with the target analyte and to provide a measurement signal indicative of the interaction of the respective sensing site (i.e. respective capture species) with the target analyte, wherein the capture species on each of the plural sensing sites is different to the capture species on the other of the plural sensing sites, and each capture species has a binding affinity for the target analyte, the binding affinity of each of the capture species being different to the other of the capture species.

Subsequently, determining the property of the target analyte 388 using measurement signals from at least two of the plural sensing sites.

Fig. 8 schematically depicts a method for determining a property of target analyte in a sample 480, the method comprising providing a sensor assembly 482, the sensor assembly comprising plural sensing sites, each of the sensing sites comprising a capture species configured to specifically bind with the target analyte and to provide a measurement signal indicative of the interaction of the respective sensing site with the target analyte, wherein the capture species on each of the plural sensing sites is different to the capture species on the other of the plural sensing sites, and each capture species has a binding affinity for the target analyte, the binding affinity of each of the capture species being different to the other of the capture species. The method further comprises determining an initial property measurement 484 based on the measurement signal from at least one sensing site of the plural sensing sites and selecting at least one sensing site of the plural sensing sites for measurement of the property 486, based on the initial property measurement. Subsequently, the method comprises determining the property of the target analyte 488 based on the measurement signal from the selected at least one sensing site.

Additionally or alternatively, the system 100 incorporating the sensor assembly 205 can be used to determine the concentration of the target analyte based on at least two of the first to fourth measurement signals. This may be used in conjunction with the step of determining the initial concentration, or may be separate. For example, an expected target analyte concentration, the control unit 160 may be configured to determine the property based on plural of the first to fourth measurement signals based on where the expected concentration falls within the respective dynamic ranges.

It should be understood that the detailed description and specific examples, while indicating exemplary embodiments of the apparatus, systems and methods, are intended for purposes of illustration only and are not intended to limit the scope. These and other features, aspects, and advantages of the apparatus, systems and methods of the present invention can be better understood from the description, appended claims or aspects, and accompanying drawings. It should be understood that the Figures are merely schematic and are not drawn to scale. It should also be understood that the same reference numerals are used throughout the figures to indicate the same or similar parts.

Other variations to the disclosed embodiments can be understood and effected by those skilled in the art in practicing the disclosure, from a study of the drawings, the disclosure, and the appended aspects or claims. In the aspects or claims, the word "comprising" does not exclude other elements or steps, and the indefinite article "a" or "an" does not exclude a plurality. The mere fact that certain measures are recited in mutually different dependent aspects or claims does not indicate that a combination of these measures cannot be used to advantage. Any reference signs in the claims should not be construed as limiting the scope.

Aspects of the invention will now be described:

Aspect 1. A system for determining a property of target analyte in a sample, the system comprising: a sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; wherein the system further comprises a control unit configured to determine the property of the target analyte; and wherein the control unit is configured to use measurement signals from at least two of the plural sensing sites in determining the property of the target analyte.

Aspect 2. The system of aspect 1, wherein the control unit being configured to use measurement signals from at least two of the sensing sites in determining the property of the analyte comprises: determining a property of the target analyte based on measurement signals from at least two of the plural sensing sites.

Aspect 3. The system of one of aspect 1 or aspect 2, wherein the control unit is configured to determine a composite baseline signal based on the measurement signal from at least two of the plural sensing sites in the absence of target analyte on the respective sensing sites; and wherein the control unit is configured to determine a property of the target analyte based on measurement signals from at least one of the plural sensing sites and the composite baseline signal.

Aspect 4. The system of any preceding aspect, wherein the control unit being configured to determine the property of the analyte comprises: determining an initial property measurement based on the measurement signal from at least a first sensing site of the plural sensing sites; selecting at least one other sensing site of the plural sensing sites for measurement of the property, based on the initial property measurement; and determining the property of the target analyte based on the measurement signal from the selected at least one other sensing site.

Aspect 5. The system of aspect 4, wherein each of the plural sensing sites has a corresponding limit of detection (LOD) and dynamic range for measuring the property of the target analyte, determined at least in part by the binding affinity of the respective capture species; and wherein selecting at least one other sensing site of the plural sensing sites for measurement of the property is based on a comparison of the initial property measurement with the LOD and/or dynamic range of the other of the plural sensing sites.

Aspect 6. The system of aspect 5, wherein the first sensing site has a first dynamic range extending from a lower concentration to an upper concentration, and wherein the respective dynamic range of each of other of the plural sensing site(s) extend from respective lower concentrations and upper concentrations falling between the lower concentration to an upper concentration of the first dynamic range.

Aspect 7. The system of one of aspect 5 or aspect 6, wherein the first sensing site has a dynamic range having a mid-point $M_1$, and wherein the sensor assembly comprises at least a second sensing site having a dynamic range with a mid-point $M_2$ and at least a third sensing site having a dynamic range with a mid-point $M_3$, and wherein $M_2 > M_1 > M_3$.

Aspect 8. The system of any one of aspects 4 to 7, wherein the capture species of the first sensing site has a rate of binding to the target analyte ($k_{on}$) which is higher than the rate of binding to the target analyte for the capture species of the other of the plural sensing sites; and/or wherein the capture species of the first sensing site has a binding affinity which is less than the binding affinity of the capture species of the other of the plural sensing sites.

Aspect 9. The system of any preceding aspect, wherein each of the capture species provided on each respective sensing site is a capture antibody and each capture antibody has a dissociation constant ($K_D$) of from $10^{-4}$ to $10^{-15}$.

Aspect 10. The system of any preceding aspect, wherein the control unit is configured to determine the property of the target analyte based on the rate of change in a measurement signal of at least one of the plural sensing sites.

Aspect 11. A method for determining a property of target analyte in a sample, the method comprising: providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; and determining the property of the target analyte using measurement signals from at least two of the plural sensing sites.

Aspect 12. The method of aspect 11, determining the property of the analyte using measurement signals from at least two of the plural sensing sites comprises: determining a property of the target analyte based on measurement signals from at least two of the plural sensing sites.

Aspect 13. The method of one of aspect 11 or aspect 12, further comprising: determining a composite baseline signal based on the measurement signal from at least two of the plural sensing sites in the absence of target analyte on the respective sensing sites; and determining a property of the target analyte based on measurement signals from at least one of the plural sensing sites and the composite baseline signal.

Aspect 14. The method of any of aspects 11 to 13, wherein the determining the property of the analyte comprises: determining an initial property measurement based on the measurement signal from at least a first sensing site of the plural sensing sites; selecting at least one other sensing site of the plural sensing sites for measurement of the property, based on the initial property measurement; and determining the property of the target analyte based on the measurement signal from the selected at least one other sensing site.

Aspect 15. The method of aspect 14, wherein each of the plural sensing sites has a corresponding limit of detection (LOD) and dynamic range for measuring the property of the target analyte, determined at least in part by the binding affinity of the respective capture species; and wherein selecting at least one other sensing site of the plural sensing sites for measurement of the property is based on a comparison of the initial property measurement with the LOD and/or dynamic range of the other of the plural sensing sites.

Aspect 16. The method of any of aspects 11 to 15, wherein determining the property of the target analyte is based on a rate of change in a measurement signal of at least one of the plural sensing sites.

Aspect 17. A system for determining a property of target analyte in a sample, the system comprising: a sensor assembly, comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; wherein the system further comprises a control unit configured to determine the property of the target analyte by: determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

Aspect 18. A method for determining a property of target analyte in a sample, the method comprising: providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; and determining the property of the target analyte by: determining an initial property measurement based on the measurement signal from at least one sensing site of the plural sensing sites; selecting at least one sensing site of the plural sensing sites for measurement of the property, based on the initial property measurement; and determining the property of the target analyte based on the measurement signal from the selected at least one sensing site.

Aspect 19. A sensor assembly, comprising: plural sensing sites, each of the sensing sites comprising a capture species configured to specifically bind with a target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species.

Aspect 20. The sensor assembly of aspect 19, wherein the plural sensing sites comprise: a first sensing site comprising a first capture species, the first capture species having a first binding affinity for the target analyte and the first sensing site providing a first measurement signal indicative of the interaction of the first capture species with the target analyte; a second sensing site comprising a second capture species having a second binding affinity for the target analyte and the second sensing site providing a second measurement signal indicative of the interaction of the second capture species with the target analyte; and a third sensing site comprising a third capture species having a third binding affinity for the target analyte and the third sensing site providing a third measurement signal indicative of the interaction of the third capture species with the target analyte.

**Claims**

1. A system for determining a property of target analyte in a sample, the system comprising:

   a sensor assembly comprising plural sensing sites, each of the sensing sites comprising a capture species configured to specifically bind with the target analyte and wherein each sensing site provides a measurement signal indicative of the interaction of the respective capture species with the target analyte, wherein the capture species on each of the plural sensing sites is different to the capture species on the other of the plural sensing sites, and each capture species has a binding affinity for the target analyte, the binding affinity of each of the capture species being different to the other of the capture species; wherein the system further comprises a control unit configured to determine the property of the target analyte; and wherein the control unit is configured to use measurement signals from at least two of the plural sensing sites in determining the property of the target analyte.

2. The system of claim 1, wherein the control unit being configured to use measurement signals from at least two of the sensing sites in determining the property of the analyte comprises:
   determining a property of the target analyte based on measurement signals from at least two of the plural sensing sites.

3. The system of claim 1 or claim 2, wherein the control unit is configured to determine a composite baseline signal based on the measurement signal from at least two of the plural sensing sites in the absence of target analyte on the respective sensing sites; and wherein the control unit is configured to determine a property of the target analyte based on measurement signals from at least one of the plural sensing sites and the composite baseline signal.

4. The system of any preceding claim, wherein the control unit being configured to determine the property of the analyte comprises:

   determining an initial property measurement based on the measurement signal from at least a first sensing site of the plural sensing sites; selecting at least one other sensing site of the plural sensing sites for measurement of the property, based on the initial property measurement; and determining the property of the target analyte based on the measurement signal from the selected at least one other sensing site.

5. The system of claim 4, wherein each of the plural sensing sites has a corresponding limit of detection (LOD) and dynamic range for measuring the property of the target analyte, determined at least in part by the binding affinity of the respective capture species; and wherein selecting at least one other sensing site of the plural sensing sites for measurement of the property is based on a comparison of the initial property measurement with the LOD and/or dynamic range of the other of the plural sensing sites.

6. The system of claim 5,

   (i) wherein the first sensing site has a first dynamic range extending from a lower concentration to an upper concentration, and wherein the respective dynamic range of each of other of the plural sensing site(s) extend from respective lower concentrations and upper concentrations falling between the lower concentration to an upper concentration of the first dynamic range; and/or

(ii) wherein the first sensing site has a dynamic range having a mid-point $M_1$, and wherein the sensor assembly comprises at least a second sensing site having a dynamic range with a mid-point $M_2$ and at least a third sensing site having a dynamic range with a mid-point $M_3$, and wherein $M_2 > M_1 > M_3$.

7. The system of any of claims 4 to 6, wherein the capture species of the first sensing site has a rate of binding to the target analyte ($k_{on}$) which is higher than the rate of binding to the target analyte for the capture species of the other of the plural sensing sites; and/or
wherein the capture species of the first sensing site has a binding affinity which is less than the binding affinity of the capture species of the other of the plural sensing sites.

8. The system of any preceding claim,

wherein each of the capture species provided on each respective sensing site is a capture antibody and each capture antibody has a dissociation constant ($K_D$) of from $10^{-4}$ to $10^{-15}$; and/or
wherein the control unit is configured to determine the property of the target analyte based on the rate of change in a measurement signal of at least one of the plural sensing sites.

9. A method for determining a property of target analyte in a sample, the method comprising:

providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte,
wherein the capture species on each of the plural sensing sites is different to the capture species on the other of the plural sensing sites, and each capture species has a binding affinity for the target analyte, the binding affinity of each of the capture species being different to the other of the capture species; and
determining the property of the target analyte using measurement signals from at least two of the plural sensing sites.

10. The method of claim 9, determining the property of the analyte using measurement signals from at least two of the plural sensing sites comprises:
determining a property of the target analyte based on measurement signals from at least two of the plural sensing sites.

11. The method of claim 9 or claim 10,

(i) wherein the method further comprises :

determining a composite baseline signal based on the measurement signal from at least two of the plural sensing sites in the absence of target analyte on the respective sensing sites; and
determining a property of the target analyte based on measurement signals from at least one of the plural sensing sites and the composite baseline signal; and/or

(ii) wherein determining the property of the target analyte is based on a rate of change in a measurement signal of at least one of the plural sensing sites.

12. The method of any of claims 9 to 11, wherein the determining the property of the analyte comprises:

determining an initial property measurement based on the measurement signal from at least a first sensing site of the plural sensing sites;
selecting at least one other sensing site of the plural sensing sites for measurement of the property, based on the initial property measurement; and
determining the property of the target analyte based on the measurement signal from the selected at least one other sensing site; and
optionally wherein each of the plural sensing sites has a corresponding limit of detection (LOD) and dynamic range for measuring the property of the target analyte, determined at least in part by the binding affinity of the respective capture species, and selecting at least one other sensing site of the plural sensing sites for measurement of the property is based on a comparison of the initial property measurement with the LOD and/or dynamic range of the other of the plural sensing sites.

13. A system for determining a property of target analyte in a sample, the system comprising:

a sensor assembly comprising plural sensing sites, each of the sensing sites comprising a capture species configured to specifically bind with the target analyte and wherein each sensing site provides a measurement signal indicative of the interaction of the respective capture species with the target analyte,
wherein the capture species on each of the plural sensing sites is different to the capture species on the other of the plural sensing sites, and each capture species has a binding affinity for the target analyte, the binding affinity of each of the capture species being different to the other of the capture species;

wherein the system further comprises a control unit configured to determine the property of the target analyte by:

determining an initial property measurement based on the measurement signal from at least one sensing site of the plural sensing sites;

selecting at least one sensing site of the plural sensing sites for measurement of the property, based on the initial property measurement; and

determining the property of the target analyte based on the measurement signal from the selected at least one sensing site.

14. A method for determining a property of target analyte in a sample, the method comprising:

providing a sensor assembly, the sensor assembly comprising plural sensing sites, each of the sensing sites comprising a capture species configured to specifically bind with the target analyte and wherein each sensing site provides a measurement signal indicative of the interaction of the respective capture species with the target analyte,

wherein the capture species on each of the plural sensing sites is different to the capture species on the other of the plural sensing sites, and each capture species has a binding affinity for the target analyte, the binding affinity of each of the capture species being different to the other of the capture species; and

determining the property of the target analyte by:

determining an initial property measurement based on the measurement signal from at least one sensing site of the plural sensing sites;

selecting at least one sensing site of the plural sensing sites for measurement of the property, based on the initial property measurement; and

determining the property of the target analyte based on the measurement signal from the selected at least one sensing site.

15. A sensor assembly, comprising:

plural sensing sites, each of the sensing sites comprising a capture species configured to specifically bind with a target analyte and wherein each sensing site provides a measurement signal indicative of the interaction of the respective capture species with the target analyte,

wherein the capture species on each of the plural sensing sites is different to the capture species on the other of the plural sensing sites, and each capture species has a binding affinity for the target analyte, the binding affinity of each of the capture species being different to the other of the capture species; and

optionally wherein the plural sensing sites comprise:

a first sensing site comprising a first capture species, the first capture species having a first binding affinity for the target analyte and the first sensing site providing a first measurement signal indicative of the interaction of the first capture species with the target analyte;

a second sensing site comprising a second capture species having a second binding affinity for the target analyte and the second sensing site providing a second measurement signal indicative of the interaction of the second capture species with the target analyte; and

a third sensing site comprising a third capture species having a third binding affinity for the target analyte and the third sensing site providing a third measurement signal indicative of the interaction of the third capture species with the target analyte.

FIG. 1

FIG. 2A

FIG. 2B

FIG. 3

FIG. 4

FIG. 5

FIG. 6

380

382

388

FIG. 7

480

482

484

486

488

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JARMOSKAITELSHRAQ et al.** How to measure and evaluate binding affinities. *eLife,* 2020, vol. 9, e57264, https://doi.org/10.7554/eLife.57264

- **YLERA.** Off-rate screening for selection of high-affinity anti-drug antibodies. *Analytical Biochemistry,* 2013, vol. 441 (2), ISSN 0003-2697, 208-213